Europäisches Patentamt

European Patent Office

Office européen des brevets

(11) Publication number : **0 494 776 A1**

(12) # EUROPEAN PATENT APPLICATION

(21) Application number : **92300171.3**

(22) Date of filing : **09.01.92**

(51) Int. Cl.⁵ : **C12N 15/00, A61K 48/00, C12N 9/12, C12N 15/38, A01K 67/00**

(30) Priority : **11.01.91 GB 9100612**

(43) Date of publication of application :
**15.07.92 Bulletin 92/29**

(84) Designated Contracting States :
**AT BE CH DE DK ES FR GB GR IT LI LU MC NL PT SE**

(71) Applicant : **UNIVERSITY OF EDINBURGH**
**Old College**
**South Bridge, Edinburgh EH8 9YL (GB)**

(72) Inventor : **Bishop, John Oliver**
**4300 N. Charles street, Apt 11-C**
**Baltimore, MD 21218 (US)**
Inventor : **Al-Shawi, Raya Anne**
**94 Warrender Park Road**
**Edinburgh, Lothian (GB)**
Inventor : **Wallace, Helen Anne Chapman,**
**AFRC Centre for**
**Genome Research Uni.of Edinburgh kings Buildings**
**West Mains Road, Edinburgh, EH9 3JQ (GB)**

(74) Representative : **Ritter, Stephen David et al**
**Mathys & Squire 10 Fleet Street**
**London EC4Y 1AY (GB)**

(54) **Method of altering the metabolic state of non-dividing cells and obtained transgenic animals.**

(57)    A method is provided for selectively converting non-mitotic cells contained in a population of animal cells from a first to a second metabolic state by contacting the cells with an activating agent. In one embodiment a transgenic animal is provided in which selected cell populations or tissues can be ablated. More specifically transgenic mice were created having the Herpes simplex virus thymidine kinase encoding gene (HSV-1-tk) coupled with the bovine thyroglobulin promotor. The thyroid follicle cells of these mice were ablated by treating the mice with Ganciclovir.

EP 0 494 776 A1

Jouve, 18, rue Saint-Denis, 75001 PARIS

This invention relates to a method of altering the metabolic state of non-dividing cells. More specifically, but not exclusively, this invention relates to methods of ablating non-dividing cells, and to therapeutic agents useful in ablating non-dividing cells. Transgenic animals and methods of producing them are further provided according to the invention.

Numerous therapeutic methods exist which aim to destroy rapidly dividing, i.e. mitotic cells. These methods, commonly used in the treatment of malignant diseases, typically involve the use of cytotoxic agents and radiation and rely upon the differential sensitivity of normal and malignant cells to these therapies.

There is however a pressing need for a controllable method for altering the metabolic state of non-dividing cells, particularly ablating selected cells by non-surgical means, which would be an extremely valuable tool in creating animal models of disease in which a target tissue is composed predominantly of non-dividing cells. The availability of such a method further would allow the development of new therapies for degenerative diseases in man and animals. Methods based on ablation of non-dividing cells could also be exploited for the treatment of those viral and parasitic diseases which are characterised by the presence of non-mitotic infected or parasitised cells.

A controllable ablation method of cells which is not dependent on DNA replication will be a valuable tool in the treatment of viral, parasitic or other states of disease.

There is also a need for animal models of common metabolic disorders characterised by metabolite and/or hormone imbalances, more specifically, the absence or over-production of specific hormones. Animal models exhibiting such metabolite and/or hormone imbalances can be produced experimentally by the surgical removal of tissues responsible for metabolite and/or hormone production.

Thus the production of animals lacking pituitary hormones can be effected by hypophysectomy and animals lacking thyroid hormones can be produced by partial or complete thyroidectomy.

However such surgical intervention is crude and inconvenient and often results in the extinction of multiple hormones. Thus two types of cells in the thyroid gland secrete hormones; the follicle cells secrete thyroxine, while the C-cells secrete calcitonin. Surgical ablation of the thyroid generally results in simultaneous loss of both types of cell, while chemical procedures, such as the administration of methimazole inhibits thyroxine production, but may cause side effects. There is accordingly a need for animal models and procedures which avoid these shortcomings.

According to one aspect of the present invention there is provided a method of selectively converting cells contained in a population of animal cells from a first to a second metabolic state, which cells contain one or more target foreign genes, which method comprises contacting said cells with an activating agent, characterised in that the cells are essentially non-mitotic and the activating agent is substantially incapable of mediating conversion to said second metabolic state of cells not containing said foreign gene or genes and/or not containing an effective promotor for at least one of said foreign gene or genes and/or not providing a metabolic environment allowing expression of at least one of said foreign genes.

According to a further aspect of the present invention there are provided transgenic animals having somatic or germ cells containing one or more target foreign genes, characterised in that the cells are essentially non-mitotic and are capable of being converted from a first to a second metabolic state on contacting said cells with an activating agent, the activating agent being substantially incapable of mediating conversion to said second metabolic state of cells not containing said foreign gene or genes and/or not containing an effective promotor for at least one of said foreign gene or genes and/or not providing a metabolic environment allowing expression of at least one of said foreign genes.

In both aspects, said cells preferably form part of a population of cells, animal tissue, animal organ or animal body that (i) includes cells not containing said one or more foreign genes and/or (ii) includes cells not containing an effective promotor for at least one of said foreign gene or genes, and/or (iii) not providing a metabolic environment allowing expression of at least one of said foreign genes, whereby said included cells (i) and/or (ii) and/or (iii) are not converted to said second metabolic state on contacting said cell population with said activating agent.

Cells which are essentially non-mitotic in accordance with the invention may be identified in a number of ways. For example they may be characterised by the presence of less than 5%, preferably less than 1% of mitotic figures. Most preferably they are characterised by the presence of less than 0.2%, preferably less than 0.1% of mitotic figures. Alternatively or aditionally, they may be characterised by the substantial absence of DNA synthesis, for example as detected experimentally by observing low or negligible levels DNA-precursor incorporation. Similarly cells containing a target foreign gene and which are essentially non-mitotic in accordance with the invention may be virus infected cells which are characterised both by substantial absence of cell division and by the essential absence of viral multiplication. Examples of such cells are non-stimulated HIV-infected T-cells.

Examples of target cells which may be converted from a first to a second metabolic state in accordance with the invention include differentiated cells, particularly of adult tissue, such as:

(a) hormone secreting cells,

(b) neuronal cells, particularly neurotransmittter and neuromodulator producing cells,

(c) receptor cells, e.g. cells capable of responding to hormonal or neurotransmitter signals.

Where the cells are hormone-secreting cells of class (a), the foreign gene may be linked to a promotor for a gene associated with hormone production whereby the activating agent may then interact with said gene associated with hormone production and production of hormone consequently regulated.

Thus for example, the foreign gene may be linked to a gene capable of expressing a hormone or hormone-precursor or to a gene capable of expressing an enzyme or enzyme precursor associated with hormone synthesis. One particular example of such a gene is the insulin gene. In this case the cells would typically be pancreatic cells. Other examples of hormone-producing cells of class (a) are thyroid cells, pituitary cells, hypothalamus cells, adrenal cells, testicular cells, ovarian cells and parathyroid cells.

Examples of neurotransmitter and neuromodulator-producing cells are cells associated with the production of dopamine and acetylcholine. Where the cells are receptor cells of class (b), e.g. neurotransmitter cells the foreign gene may be operatively linked to a promotor gene for a neurotransmitter protein.

Where the cells are receptor cells, e.g. cells of class (c) above, the foreign gene may be linked to the receptor gene whereby the activating agent interacts with the receptor gene and the latter regulates expression of the foreign gene.

Many examples of different first and second metabolic states are envisaged in accordance with the invention. One such state is the normal metabolic state of the cells. However as indicated it is essential for the cells in said first metabolic state to be essentially non-dividing and it is preferred for the cells in said second metabolic state also to be essentially non-dividing. In one specific embodiment of the invention said second metabolic state comprises death or ablation of said cells, i.e. conversion to a metabolic state wherein cell function ceases.

Conveniently, the presence of cells in said second metabolic state is detectable by assaying a metabolite produced (and particularly secreted) by cells in one but not another of said first and second metabolic states. For example cells in said first metabolic state, but not in said second metabolic state, may produce a characteristic protein or other substance, especially ahormone. Detection of said hormone can thus be used to detect conversion of cells to the second metabolic state.

Alternatively, animals having a substantial absence of cells in the first metabolic state can be used as an experimental model for defficiencies of the hormone and are thus useful in the development of drugs for countering such deficiencies.

Conveniently said activating agent is a substrate of an enzyme, the enzyme, or a precursor thereof being coded for by said foreign gene. In this embodiment, the substrate may be convertible, in the presence of said enzyme, to a cytotoxic substance.

In exemplary embodiments of the invention said foreign gene can code for a nucleoside kinase, for example a thymidine kinase. For example, the foreign gene might code for a Herpes simplex thymidine kinase.

Examples of activating substance which are substrates for thymidine kinase include 9-[1,3-dihydroxy-2-propoxy)-methyl]-guanine (Ganciclovir) and [1-(2-deoxy-2-fluoro-$\beta$-D-arabino-furanosyl)-5-iodouracil] (FIAU).

The somatic cells containing one or more target foreign genes may be thyroid follicle cells which are ablated on administration of said activating agent.

Transgenic animals, the thyroid follicle cells of which are capable of being selectively ablated on administration to said animal of an activating agent are novel and form a further aspect of the invention.

The present invention has further application in the field of therapy and diagnostics.

Thus according to a further aspect of the invention there is provided the use of a recombinant DNA molecule coding for a nucleoside kinase in the manufacture of a therapeutic agent for ablating non-mitotic cells by a procedure comprising targetting said non-mitotic cells with said therapeutic agent and contacting said non-mitotic cells with a substrate for the nucleoside kinase which is convertible to a cytotoxic substance , wherein said therapeutic agent comprises a combination of said recombinant DNA molecule coupled with a promotor.

The invention further provides the use of a substrate for a nucleoside kinase which substrate is convertible to a cytotoxic substance, in the manufacture of a therapeutic agent for ablating non-mitotic cells.

Such use may comprise targetting said non-mitotic cells with a combination of a recombinant DNA molecule coding for a nucleoside kinase coupled with a promotor, and contacting said non-mitotic cells with said substrate for the nucleoside kinase.

The invention further provides a method for producing a transgenic animal having somatic or germ cells containing one or more target foreign genes, characterised in that the cells are essentially non-mitotic and are capable of being converted from a first to a second metabolic state on contacting said cells with an activating agent, which activating agent is incapable of mediating conversion of cells not containing said foreign gene to said second metabolic state, and/or cells not containing an effective promotor for at least one of said foreign gene or genes and/or not providing a metabolic environment allowing expression of at least one of said foreign genes, which comprises the steps of (i) coupling a recombinant DNA molecule comprising said foreign gene with a promotor active in said animal to form a coupled recombinant DNA molecule, (ii) introducing said coupled product into the animal cells. Preferably such method further comprises (iii) selecting animal progeny demonstrating the desired transgenic phenotype.

A variation of this method which forms a further aspect of the invention comprises producing a cell population comprising cells containing one or more target foreign genes, characterised in that the cells are essentially non-mitotic and are capable of being converted from a first to a second metabolic state on contacting said cells with an activating agent, which activating agent is incapable of mediating conversion of cells not containing said foreign gene to said second metabolic state, and/or not containing an effective promotor for at least one of said foreign gene or genes and/or not providing a metabolic environment allowing expression of at least one of said foreign genes, which comprises the steps of (i) coupling a recombinant DNA molecule comprising said foreign gene with a promotor active in said animal to form a coupled recombinant DNA molecule, (ii) introducing said coupled product into the cells.

In each of these variants, preferably the promotor is active only in selected target cells.

The introduction of foreign DNA into animal cells in accordance with the invention may be achieved in a variety of ways, including the following known procedures which are commonly used in the production of transgenic animals:

(i) direct injection into fertilised eggs,

(ii) infection of embryonic cells with a virus, e.g. a retrovirus,

(iii) injection of ES cells (embryonic stem cells) into a blastocyst.

Further in accordance with the invention there is provided a method of ablating non-dividing cells, which comprises contacting said cells with an activating agent which is a substrate of an enzyme, said enzyme, or a precursor thereof being coded for by a target foreign gene in the genome of said cells and wherein the enzyme is capable of converting the activating agent to a cytotoxic substance.

As indicated, one embodiment of the invention is based upon the use of a cell containing a viral gene product capable of converting an activating agent to a cytotoxic form. In another embodiment, a cell expressing a foreign gene product, e.g. a viral gene product may be targetted with an antibody to that gene product. The antigen-antibody interaction may be used to detect targetted cells or to assist in their destruction. In an example of the first of these embodiments, Herpes simplex virus thymidine kinase encoding gene (HSV1tk) which is coupled with a suitable cell-specific promoter and targeted to offending cells such as non-dividing HIV-1 or HIV-2 infected T-cells; treatment with GCV will eradicate those cells. The method may also be used in controlling other conditions where conventional treatments depend on active cell division.

Herpes Simplex virus thymidine kinase (HSV1TK) is able to phosphorylate a number of natural pyrimidines and some synthetic purine and pyrimidine nucleoside analogues. When the gene encoding this kinase (HSV1tk) is introduced into and expressed in cells, normal cell function is maintained. However, on treating the cells with specific nucleoside analogues such as 9-[1,3-dihydroxy-2-propoxy)-methyl]-guanine (Gancyclovir) and [1-(2-deoxy-2-fluoro-$\beta$-D-arabino-furanosyl)_5-iodouracil] (FIAU). Phosphorylation of these compounds by HSV1TK leads to cell death.

According to this invention, this technology has been used in to create transgenic rodents, specifically mice and found to be successful in specifically ablating selected cell populations or tissues. The application of the invention to other species, particularly mammalian species is also encompassed.

Hitherto, it has been assumed that ablation of cells by HSV1TK is achieved by incorporation of the nucleoside triphosphates into DNA and that cell division is therefore a prerequisite for achieving cell death. Indeed, all experiments reported to date have used dividing cells as their ablation target. Our data however show that it is also possible to ablate non-dividing cells. Ablation of non-dividing cells, the subject of this patent application, will be a valuable tool in research and medicine.

One particularly useful application of the invention is to enable the production of animal models having hormone levels altered from those ocurring in normal animals. For example the invention has enabled the production of thyroxine-deficient animals.

The thyrocytes of the thyroid gland express thyroglobulin, the precursor of thyroxine. The normal adult thyroid gland is basically a non-dividing tissue and mitotic figures in adult mouse thyrocytes are extremely rare (less than 1:1000). (Herein the terms non-mitotic and non-dividing are used synonymously to refer to cells wherein the mitotic figures are less than 1:20, preferably less than 1:100, most preferably less than 1:500). Examples of such said cells are cells of a morphologically distinct organ, as opposed to populations of cells in the circulation or lymphatic system. Other examples include non-dividing cells in the circulation, e.g. non-dividing T-cells.

In our experiments, we targeted expression of HSV1TK to the thyrocytes, by linking the bovine thyroglobulin promoter to the HSV1tk gene and introducing the hybrid gene into the germ-line of mice. Transgenic mice developed normally and HSV1TK in female mice was found to be expressed only in the thyroid. Upon treatment of adult mice with the anti-herpetic drug GCV, a complete loss of thyroxine was observed within 14 days. Levels of another thyroid hormone, calcitonin, and a hormone produced by a gland intimately associated with the thyroid, parathyroid hormone, were only reduced moderately if at all. This shows that the deleterious effects of phosphorylation of GCV by HST1TK are restricted to the cells expressing the foreign gene. In case mitosis might have been active in thyroids of transgenic mice or transgenic mice treated with GCV we examined thyroid sections for mitotic figures, but detected no difference from un-treated non-transgenic mice. A more detailed description of the experimental work follows.

Two cell types in the thyroid gland secrete hormones [1]: the follicle cells secrete thyroxine while the C-cells secrete calcitonin. Surgical ablation of the thyroid results in the simultaneous loss of both cell types, while the administration of methimazole inhibits thyroxine production variably and may cause side effects. The genetically hypothyroid mouse suffers from secondary defects due to thyroxine deficiency during development [2,3] and retains a significant level (10%) of circulating thyroxine [4]. To achieve complete withdrawal of thyroxine without complicating side effects, we targeted herpes simplex thymidine kinase to the follicle cells of transgenic mice by linking the tk gene to the bovine thyroglobulin gene promoter. Upon treatment with the antiherpetic agent Ganciclovir a complete loss of thyroxine was observed within 14 days. Levels of calcitonin and parathyroid hormone were reduced only moderately. No recovery of thyroxine production was observed following withdrawal of the ablating agent. The synthesis and secretion of the mouse major urinary proteins (MUP) in the liver is dependent on circulating thyroxine[5]. MUP synthesis ceased in the transgenically ablated mice, and was completely restored by administration of T4. Since the treated mice were adults (up to 18 weeks of age), total or partial thyroid ablation may be performed at any age following completely normal development.

The results show that this method of ablation may be applicable not only to dividing cells, but also to cell types with low mitotic activity.

Previously, expression of the herpes virus thymidine kinase (HSV1-tk) gene was targeted to the lymphocytes with a combination of the tissue-specific immunoglobulin $\mu$ enhancer and kappa promoter elements[6]. Treatment with either of two antiherpetic agents 9-[1,3-dihydroxy-2-propoxy)methyl]-guanine (Gancyclovir) and [1-(2-deoxy-2-fluoro-$\beta$-Darabino-furanosyl)-5-iodouracil] (FIAU) led to extensive loss of spleen and thymus tissue and of both B and T-lymphocytes[6,7]. Similarly, expression of the herpesvirus gene was targeted to the somatotrophs with the growth hormone gene promoter (construct GHtk). In mice transgenic for the GHtk gene, treatment with FIAU led to complete ablation of both somatotrophs and lactotrophs, without impairment of other specialised secretory pituitary cells such as thyrotrophs and gonadotrophs[8]. In these experiments ( where the authors treated fetuses *in utero* in order to ablate populations of dividing cells) lymphocytes and pituitary somatotrophs and lactotrophs regenerated when the antiherpetic agent was withdrawn.

The potential of transgenic ablation according to the invention is considerable. Two main desiderata are that the ablating agent should not be toxic in its own right or be converted to a toxic product by cellular enzymes in the absence of the herpes virus thymidine kinase (HSV1-TK), and that HSV1-TK should not affect the cells in the absence of the antiherpetic agent. FIAU and GCV were developed, like Acyclovir, to combat herpes virus infections. They are efficacious first because they are non-toxic in their unaltered state but become toxic when phosphorylated, and secondly because they are efficiently phosphorylated by HSV1-TK but less efficiently or not at all by cellular nucleoside kinases. The only physiological effect of transgenic HSVI-tk genes so far reported is that they tend to be expressed in the testis irrespective of the nature of the promoter to which they are linked, and cause male sterility (Ref. 9 and unpublished observations).

Additional desirable properties are that the phosphorylation products of the antiherpetic agent employed should not pass between cells, and that both dividing and differentiated target cells should be killed.

An ablation system with these characteristics might be applied to specific cell lineages during development, or to specific cell types in complex adult organs such as the brain. The thyroid is an appropriate organ in which to explore the possibilities of ablation. It contains two types of secretory cells, the follicle cells and the C-cells which secrete calcitonin, and is in intimate contact with the parathyroid gland which secretes parathyroid hormone. In the adult the follicle cells are essentially non-dividing but can be stimulated to divide by supranormal levels of TSH[10]. Thyroglobulin is exclusively synthesized in follicle cells, and the promoter of the thyroglobulin gene entrains the tissue-specific expression of the chloramphenicol acetyl transferase reporter gene[11].

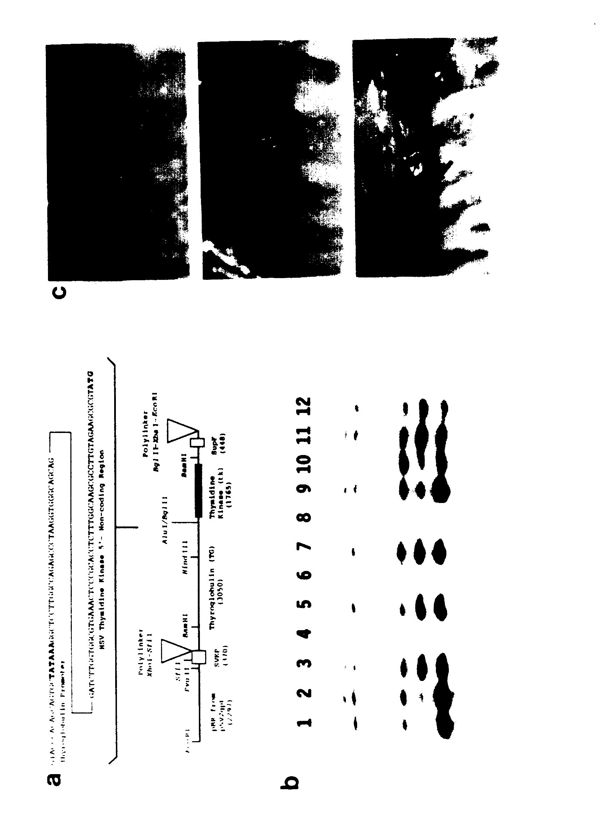

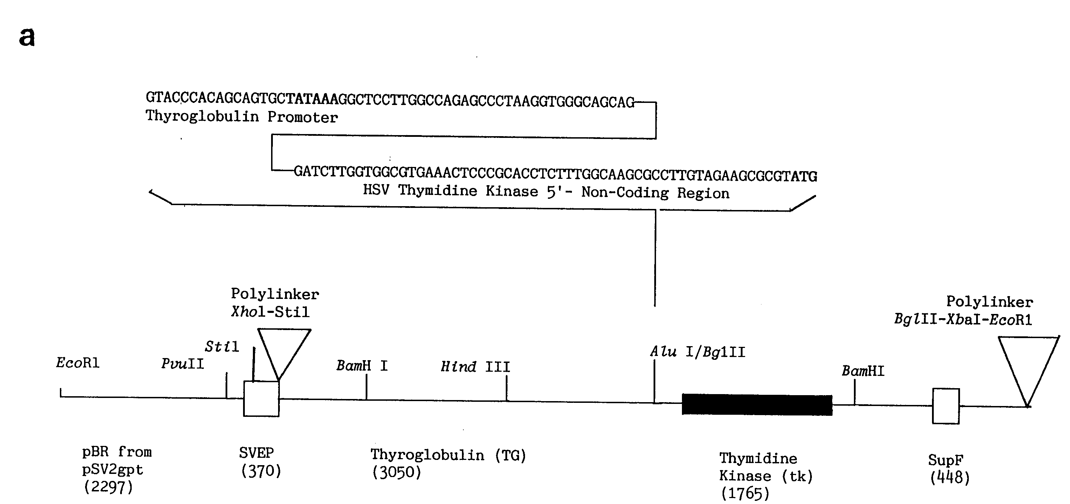

We attached the coding, 3'-non-coding and 3'-flanking region of the HSV1-tk gene to a 3.1-kb fragment containing the promoter and flanking region of the bovine thyroglobulin gene through a junction in the 5'-non-coding regions of both genes (Figure 1), and introduced the hybrid gene into the germline of mice by direct microinjection. Four lines of transgenic mice were established, three of which expressed HSV1-TK in the thyroid gland at different levels. In females, expression was confined to the thyroid. The experiments described here were performed with a line (TH66.19) established from the $G_O$ female which showed the highest level of thyroid HSV1-TK. The high expression level has been transmitted through 3 subsequent generations.

When transgenic female mice of Line TG66.19 were treated with GCV by minipump for two weeks, levels of T4 and T3 fell to below the limit of detetion of the assay, (Tables 1 and 2). The levels observed in non-transgenic mice treated with GCV in the same way were variably reduced down to a minimum of about 50%. Transgenic mice infused with saline gave a similar response. The reason for this is not known. However, the levels of T4 and T3 in untreated control mice were quite variable (Table 1), and in fact the levels observed in a mouse on different occasions were as variable as levels in different mice (data not shown).

Administration of T4 throughout the last five days of GCV treatment restored the level of circulating T3 to about half the normal level, that is, to a level similar to those observed in the controls. As would be expected, administrations of growth hormone during the second week of treatment has no effect on circulating T4 or T3 levels. The thyroid glands of GCV-treated mice were vastly reduced in size. (Fig. 1C), and this was reflected in the yield of protein obtained from them. Total soluble protein obtained from treated thyroids was less than 10% that from controls (Table 1). The total HSV-TK activity in untreated thyroid was 6 to 17.5 units in different experiments. No HSV-TK was observed in any of the treated animals, suggesting that treatment had completely destroyed the follicle cells. Thus destruction of follicle cells appears to be the main cause of the loss of circulating T4 and T3.

The parathyroid gland, which is physically associated with the thyroid, produces parathyroid hormone. No significant changes in levels of parathyroid hormone were observed in either transgenic or non-transgenic animals treated with GCV (Table 1). The production and secretion of PTH clearly do not depend upon circulating T4 or T3. Furthermore, the products of GCV phosphorylation or any other putative agents of ablation have not passed significantly to the parathyroid. Calcitonin is produced in the C-cells of the thyroid, which lie between the follicle cells.

Levels of calcitonin were measured in a second experiment (Table 2), and were found to be more than 50% of the levels in control mice infused with buffer. In view of the overall structural changes that must accompany the shrinkage of the thyroid it is perhaps surprising that calcitonin levels did not fall further. The results suggest that there is little or no transfer of phosphorylated GCV between follicle cells and C-cells. Furthermore it is unlikely that a 50% reduction in calcitonin has deleterious effects on physiology.

Transcription of the mouse major urinary protein (MUP) genes in the liver is known to be dependent on both thyroxine and growth hormone (Ref. 5 and our unpublished results). Since one of the pleiotropic functions of thyroxine is to stimulate the transcription of the growth hormone gene[12,13] and another may be to potentiate GH receptors[14], thyroxine deprivation influences MUP transcription both directly and via growth hormone. Since the MUP (proteins) are secreted from the liver and rapidly excreted into the urine, they provide an essentially non-interventionist method of estimating the hormonal status of mice. Figure 1B shows that treatment of TG-tk transgenic mice with GCV nearly eliminated the excretion of MUP proteins. MUP excretion was restored by administering thyroxine during the second week of GCV treatment. MUP excretion was normal in control transgenic mice given phosphate buffered saline by minipump and in non-transgenic mice treated with GCV. These results are thus in complete agreement with the hormonal assays, and add the further important finding that the effects of ablation on MUP synthesis and secretion were reversed by thyroxine.

In the transgenic ablation of lymphocytes and somatotrophs, repopulation of the tissue was observed following withdrawal of the antiherpetic agent[7,8]. However, when GCV was withdrawn from treated TG-tk mice there was no recovery of circulating T3 or T4, no increase in thyroid soluble protein, no recovery of HSV-TK activity in thyroid extracts and no return of MUP excretion over a period of 30 days (data not shown).

It appears that there is no thyroid stem-cell population in adult mice, and this would be consistent with thyroid regeneration through division of follicle cells. We cannot exclude alternative explanations at this time, for example expression of HSV-TK in stem cells and consequent killing by GCV, but such explanations are less likely.

At eighteen weeks of age the thyroid gland is essentially a non-dividing tissue. Mitotic figures are rare in thyroid glands of normal mice[15]. The successful ablation of the follicle cells therefore casts doubt upon the assumption generally made that phosphorylated GCV kills cells exclusively by being incorporated into DNA and preventing further extension of the polynucleotide chain. In case mitosis might have been active in thyroids of transgenic mice or transgenic mice treated with GCV we examined thyroid sections for mitotic figures, but detected no difference from untreated non-transgenic mice (data not shown). Since GCV is an analogue of guanosine, and different guanosine phosphate derivatives play several key roles in cellular metabolism, it is possible that several different lethal mechanisms come into play.

TABLE 1. Effect of GCV treatment on levels of circulating T4, T3 and PTH and on thyroid protein and
HSV-TK in TG-tk transgenic mice.

| TREATMENT | MOUSE | TOTAL PROTEIN (µg) | TOTAL TK UNITS | T4, nmol/l | | T3, nmol/l | | PTH, pmol/l | |
|---|---|---|---|---|---|---|---|---|---|
| | | AFTER | AFTER | BEFORE | AFTER | BEFORE | AFTER | BEFORE | AFTER |
| Saline | TG66.19.40 | 144 | 17.50 | 44.1 | 39.0 | | 0.58 | 50 | 45 |
| GCV | TG66.19.31 | 10 | 0.00 | 29.8 | 0.0 | | ND | 65 | 55 |
| GCV + D | TG66.19.25 | 20 | 0.00 | | | | | | |
| GCV + T4 | G3/TG66.19 | 14 | 0.00 | 50.1 | 64.2 | 0.95 | 0.33 | ND | 60 |
| GCV + T4 | TG66.19.35 | 3 | 0.00 | | | | | | |
| GCV + GH | G3/TG66.19 | 10 | 0.00 | 20.1 | 0.0 | | 0.00 | 55 | 65 |
| GCV + GH | TG66.19.32 | 7 | 0.00 | | | | | | |
| GCV | Controls | 170 | 0.00 | 21.1 | 10.8 | | 3.9 | 55 | 75 |
| None | Controls | | | $45.8 \pm 19.0$ SD (n=20) | | $0.55 \pm 0.22$ SD (n=15) | | | |

Controls were $F_2$ mice from a C57BL x CBA/Ca cross, equivalent in background to the transgenic mice. Five
mice wer sampled on 4 (T4) and 3 (T3) occasions. Transgenic mice and treatments are described in
Fig. 1. Serum samples were pooled for hormone assays as indicated. ND, not determined. Total protein and
total TK units (pmol TMP synthesised per minute) refer to thyroid extracts. T4 and T3 levels were determined
using Amerlite competitive ELISA assays (Amersham). Parathyroid hormone was determined by RIA (Amersham).

EP 0 494 776 A1

TABLE 2.   Effect of GCV treatment on levels of circulating T4, T3 and calcitonin in TG-tk transgenic mice.

| TREATMENT | MOUSE | T4, nmol/l | | T3, nmol/l | | CALCITONIN pg/l |
|---|---|---|---|---|---|---|
| | | BEFORE | AFTER | BEFORE | AFTER | AFTER |
| Saline | TG66.19.16 | 44.9 | 15.8 | ND | ND | 40 |
| Saline | TG66.19.21 | 44.0 | 24.0 | 0.86 | 0.27 | |
| GCV 3mg/d | TG66.19.23 | 52.3 | 0.0 | 0.96 | 0.00 | |
| GCV 3mg/d | G3/TG66.19 | 51.0 | 0.0 | 0.98 | 0.00 | 25 |
| GCV 4.5mg/d | TG66.19.26 | 50.7 | 0.0 | 0.83 | 0.00 | |
| NONE | Controls (2) | ND | ND | ND | ND | 50, 45 |

Calcitonin levels of two control mice are shown.  Calcitonin was measured by RIA (Amersham).

Other conditions are as for Table 1 and Fig. 1.

EP 0 494 776 A1

The method of the invention was further demonstrated by introducing the HSV-1-tk gene into NIH3T3 fibroblast cells. Specifically, clones of NIH3T3 cells which express HSV-1 thymidine kinase were derived by transfection. Two types of clone were derived. Type 1 clones carry a hybrid gene in which the mouse α-actin promoter directs the transcription of the coding region of the HSV-1-tk gene. Type 2 clones carry the HSV-1-tk gene under the control of its own promotier. HSV-1 thymidine kinase activity was present in both proliferating and growth-inhibited cells of both types.

The sensitivity of the cells to the antiherpetic agent Ganciclovir was tested when growth was inhibited by contact-inhibition. Lack of growth was confirmed by counting cells and lack of DNA synthesis by measuring thymidine incorporation into DNA. Control and experimental plates were seeded with equal numbers of cells and incubated in normal medium until confluent, when contact-inhibition prevents further cell division. Ganciclovir was then added to the experimental plates. Cell survival was measured three days later by comparing the number of cells present on the experimental plates with the number of cells present on the control plates. The results obtained show that NIH3T3 cells that contain HSV-1 thy6midine kinase activity are more sensitive to killing by Ganciclovir than NIH3T3 cells that do not contain HSV-1 thymidine kinase activity (Table 3).

Table 3. Three-day Survival of Normal HIH3T3 Cells and NIH3T3 Cells That Contain HSV-1 Thymidine Kinase Activity in the Presence of Ganciclovir.

| CELL TYPE | CONDITIONS | CELL SURVIVAL (%) |
|---|---|---|
| Normal NIH3T3 | 100 µM Ganciclovir, 3 days | 108 |
| Type 1 clone 8 | 30 µM Ganciclovir, 3 days | 23 |
| Type 2 clone 3 | 30 µM Ganciclovir, 3 days | 72 |

[REFERENCES]

1. DeGroot, L.J. *Endocrinology*, 2nd Ed. (W.B. Saunders, Philadelphia, 1989).
2. Noguchi, T. & Sugasaki, T.J. *Neurochem.* 42, 891-893 (1984).
3. Adams, P.M., Stein, S.A., Palnitkar, M., Anthony, A., Gerrity, L. & Shanklin, D.R. *Neuroendocrinol.* 49, 138-143 (1989).
4. Stein, S.A., Shanklin, D.R., Krulich, L., Roth, M.G., Chubb, C.M. & Adams, P.M. *Neuroendocrinol.* 49, 509-519 (1989).
5. Knopf, J.L., Gallagher, J.F. & Held, W.A. *Mol. Cell. Biol.* 3, 2232-2240 (1983).
6. Borrelli, E., Heyman, R., Hsi, M. & Evans, R.M. *Proc. Natl. Acad. Sci., USA 85*, 7572-7576 (1988).
7. Heyman, R.A., Borrelli, E., Lesley, J., Anderson,D., Richman, D.D., Baird, S.M., Hyman, R. & Evans, R.M. *Proc. Natl. Acad. Sci., USA 86*, 2698-2702 (1989).
8. Borrelli, E., Heyman, R.A., Adrias, C., Sawchenko, P.E. & Evans, R.M. *Nature 339*, 538-541 (1989).
9. Al-Shawi, R., Burke, J., Jones, C.T., Simons, J.P. & Bishop, J.O. *Mol. Cell. Biol. 8*, 4821-4828 (1988).
10. Sterling, L. & Lazarus, J.H. *Ann. Rev. Physiol. 39*, 349-371 (1977).
11. Ledent, C., Parmentier, M. & Vassart, G. *Proc. Natl. Acad. Sci., USA 87*, 6167-6180 (1990).
12. Evans, R.M. Birnberg, M.C. & Rosenfeld, M.G. *Proc. Natl. Acad. Sci., USA 79*, 7659-7663 (1982).
13. Spindler, S.R., Mellon, S.H. & Baxter, J.D. *J. Biol. Chem. 237*, 11627-11634 (1982).
14. Hochberg, Z., Bick, T. & Harel, Z. *Endocrinology 126*, 325-329 (1990).
15. Wollman, S.H., Andros, G., Cannon, G.B. & Eagleton, G.B., *Thyroid Neoplasia* (eds Young, S. & Inman, D.R.) 201-20 (Academic Press, New York, 1990).

**FIGURE 1**

a. Diagram of the thyroglobulin promoter-HSV thymidine kinase reporter construct (TG-tk). The sequence

of the *AluI*-*BglI*I junction between the TATA box and the HSV-*tk* initiation codon is shown.

*b*. Isoelectric focusing gel of mouse urine samples collected before and after treatment. Odd number lanes, before treatment, even number lanes, after treatment. Lanes 1 to 10, TG66.19 transgenic mice as follows: lanes 1 and 2, saline infusion; lanes 3 and 4, GCV infusion; lanes 5 and 6, GCV infusion, injection with T4 diluent; lanes 7 and 8, GCV infusion, injection with GH; Lanes 9 and 10, GCV infusion, injection with T4; lanes 11 and 12, non-transgenic control infused with GCV. Variation in the MUP pattern between individual mice is due to heterozygosity at the MUP locus in the C57/BL x CBA cross.

*c*. Thyroid glands of normal mouse (top), untreated transgenic mouse (centre) and transgenic mouse treated with 4.5mg/day GCV for 14 days (right). In each case the right thyroid gland (arrow) is exposed above the trachea.

## METHODS.

*a*. The fragment microinjected into mouse one-cell embryos was removed from the plasmid by restriction in the polylinkers (*XhoI*-*KpnI*), and includes the bacterial *SupF* gene located 3' to the *HSV-TK* reporter. The fragment was injected at a concentration of 1.5µg/ml into either pronucleus of C57BL/6 x CBA/Ca $F_2$ embryos[9]. 255 embryos were transferred to pseudopregnant recipients, 90 pups born, 15 transgenics identified and 4 lines established.

*b*. 20µl of dialysed urine was applied to each lane of an agarose gel containing pH 3.5-4.9 ampholines (Pharmacia). Following focusing (15 kVa, 90 min) the gel was stained with Coumassie blue. Transgenic female mice, all from line TG66.19, were 12 to 18 weeks of age when minipumps (Alzet mode 2002) were implanted. Ganciclovir was administered at a rate of 4.5 mg/day for 14 days. T4 (5µg/20g body weight) was injected daily for the last five days. D represents the thyroxine diluent. Bovine growth hormone (GH, 100µG/20g) was injected at 12 hour intervals for 5 days.

## Claims

1. A method of selectively converting cells contained in a population of animal cells from a first to a second metabolic state, which cells contain one or more target foreign genes, which method comprises contacting said cells with an activating agent, characterised in that the cells are essentially non-mitotic and the activating agent is substantially incapable of mediating conversion to said second metabolic state of cells not containing said foreign gene or genes and/or not containing an effective promotor for at least one of said foreign gene or genes and/or not providing a metabolic environment allowing expression of at least one of said foreign genes.

2. A method according to Claim 1 wherein said cells form part of a population of cells, animal tissue, animal organ or animal body that (i) includes cells not containing said one or more foreign genes and/or (ii) includes cells not containing an effective promotor for at least one of said foreign gene or genes and/or (iii) includes cells not providing a metabolic environment allowing expression of at least one of said foreign gene whereby said included cells (i) and/or (ii) and/or (iii) are not converted to said second metabolic state on contacting said cell population with said activating agent.

3. A method according to Claim 1 or Claim 2 wherein said cells containing one or more foreign target genes are transgenic animal cells.

4. A method according to any preceding claim wherein the proportion of cells displaying mitotic figures is less than 0.2%, preferably less than 0.1%.

5. A method according to any of Claims 1 to 4 wherein said second metabolic state comprises loss of cell function or death, or comprises ablation of said cells.

6. A method according to any preceding claim wherein the presence of cells in said second metabolic state is detectable by assaying a metabolite produced by cells in one but not another of said first and second metabolic states.

7. A method according to any preceding claim wherein cells in said first and second metabolic states produce different levels of a selected metabolite.

EP 0 494 776 A1

8. A method according to Claim 7 wherein cells in said second metabolic state produce essentially none of said metabolite.

9. A method according to any of Claims 6 to 8 wherein said metabolite is a product secreted by said cells, for example a protein, hormone or a neurotransmitter.

10. A method according to any preceding claim wherein said activating agent is a substrate of an enzyme, said enzyme, or a precursor thereof being coded for by said foreign gene, said substrate preferably being convertible, in the presence of said enzyme, to a cytotoxic substance.

11. A method according to any preceding claim wherein at least one of said foreign gene or genes is operatively linked to a promotor and said selected cells provide a metabolic environment allowing expression of said foreign gene under control of said promotor.

12. A method according to any preceding claim wherein said foreign gene codes for a protein which interacts with the activating agent to produce a product, for example an enzyme, which converts the cells from said first to said second metabolic state.

13. A method according to any preceding claim wherein said foreign gene codes for a nucleoside kinase, especially thymidine kinase, for example a Herpes simplex virus thymidine kinase.

14. A method according to any of Claim 12 or Claim 13 wherein said activating substance is selected from 9-[1,3-dihydroxy-2-propoxy)-methyl]-guanine (Ganciclovir) and [1-(2-deoxy-2-fluoro-β-D-arabino-furanosyl)-5-iodouracil] (FIAU).

15. A method according to any preceding claim wherein said cells containing one or more target foreign genes are cells of a morphologically distinct organ.

16. A method according to any of Claims 1 to 15, wherein said cells containing said one or more foreign genes are cells of the lymphatic system, for example lymphocytes, or are thyroid follicle cells.

17. A method according to Claim 16 wherein said thyroid follicle cells are ablated on administration of said activating agent.

18. A transgenic animal having somatic or germ cells containing one or more target foreign genes, characterised in that the cells are essentially non-mitotic and are capable of being converted from a first to a second metabolic state on contacting said cells with an activating agent, the activating agent being substantially incapable of mediating conversion to said second metabolic state of cells not containing said foreign gene or genes and/or not containing an effective promotor for at least one of said foreign gene or genes and/or not providing a metabolic environment allowing expression of at least one of said foreign genes.

19. A transgenic animal according to Claim 18 wherein said cells form part of a population of cells, animal tissue, animal organ or animal body that (i) includes cells not containing said one or more foreign genes and/or (ii) includes cells not containing an effective promotor for at least one of said foreign gene or genes and/or (iii) includes cells not providing a metabolic environment allowing expression of at least one of said foreign genes whereby said included cells (i) and/or (ii) and/or (iii) are not converted to said second metabolic state on contacting said cell population with said activating agent.

20. A transgenic animal according to Claim 18 or 19 wherein said second metabolic state and/or proportion of mitotic figures, and/or activating agent, and/or substrate, and/or foreign gene or genes, and/or cells is or are as defined in one or more of Claims 1 to 18.

21. The use of a recombinant DNA molecule coding for a nucleoside kinase in the manufacture of a therapeutic agent for ablating non-mitotic cells by a procedure comprising targetting said non-mitotic cells with said therapeutic agent, wherein said therapeutic agent comprises a combination of said recombinant DNA molecule coupled with a promotor, and contacting said non-mitotic cells with a substrate for the nucleoside kinase which is convertible to a cytotoxic substance.

11

**22.** The use of a substrate for a nucleoside kinase which is convertible to a cytotoxic substance, in the manufacture of a therapeutic agent for ablating non-mitotic cells.

**23.** The use claimed in Claims 21 or 22, wherein said procedure comprises targetting said non-mitotic cells with a combination of (i) recombinant DNA molecule coding for a nucleoside kinase coupled with (ii) a promotor, and contacting said non-mitotic cells with said substrate for the nucleoside kinase.

**24.** The use claimed in Claim 23 wherein said substrate is selected from:
9-[1,3-dihydroxy-2-propoxy)-methyl]-guanine (Ganciclovir) and [1-(2-deoxy-2-fluoro-β-D-arabino-furanosyl)-5-iodouracil] (FIAU).

**25.** A method for producing a transgenic animal having somatic or germ cells containing one or more target foreign genes, characterised in that the cells are essentially non-mitotic and are capable of being converted from a first to a second metabolic state on contacting said cells with an activating agent, which activating agent is incapable of mediating conversion of cells not containing said foreign gene to said second metabolic state and/or not containing an effective promotor for at least one of said foreign gene or genes and/or not providing a metabolic environment allowing expression of at least one of said foreign genes, which comprises the steps of (i) coupling a recombinant DNA molecule comprising said foreign gene with promotor active in said animal to form a coupled recombinant DNA molecule, (ii) introducing said coupled product into the animal cells.

**26.** A method for producing a cell population comprising cells containing one or more target foreign genes, characterised in that the cells are essentially non-mitotic and are capable of being converted from a first to a second metabolic state on contacting said cells with an activating agent, which activating agent is incapable of mediating conversion of cells not containing said foreign gene to said second metabolic state and/or not containing an effective promotor for at least one of said foreign gene or genes and/or not providing a metabolic environment allowing expression of at least one of said foreign genes, which comprises the steps of (i) coupling a recombinant DNA molecule comprising said foreign gene with a promotor active in said cells to form a coupled recombinant DNA molecule, (ii) introducing said coupled product into the cells.

**27.** A method of ablating non-dividing cells, which comprises contacting said cells with an activating agent which is a substrate of an enzyme, said enzyme or a precursor thereof being coded for by a target foreign gene in the genome of said cells and wherein the enzyme is capable of converting the activating agent to a cytotoxic substance.

**28.** A transgenic animal which is deficient in thyroxine.

**29.** A transgenic animal according to Claim 28 which has less than 50% of the thyroxine level of normal animals, preferably less than 20%, most preferably less than 5% of the thyroxine level of normal animals.

**30.** A transgenic animal according to Claim 29 which is substantially completely deficient in thyroxine.

**31.** A transgenic animal according to Claims 29 or 30 which is not substantially deficient in calcitonin.

**32.** A transgenic animal according to any of Claims 29 to 31 which is not substantially deficient in parathyroid hormone.

**a**

```
GTACCCACAGCAGTGCTATAAAGGCTCCTTGGCCAGAGCCCTAAGGTGGGCAGCAG
Thyroglobulin Promoter
```

```
GATCTTGGTGGCGTGAAACTCCCGCACCTCTTTGGCAAGCGCCTTGTAGAAGCGCGTATG
HSV Thymidine Kinase 5'- Non-Coding Region
```

Polylinker
*Xho*I-*Sti*I

Polylinker
*Bgl*II-*Xba*I-*Eco*R1

*Sti*I

*Alu* I/*Bgl*II

*Eco*R1          *Pvu*II                    *Bam*H I        *Hind* III                                              *Bam*HI

pBR from                SVEP              Thyroglobulin (TG)                    Thymidine                SupF
pSV2gpt                 (370)             (3050)                               Kinase (tk)              (448)
(2297)                                                                         (1765)

European Patent
Office

# EUROPEAN SEARCH REPORT

Application Number

EP    92 30 0171

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 334 301 (VIAGENE, INC.)<br><br>* page 23, column 1, line 32 - page 30, column 2, line 41; claims 1,9,10,12,18,20,21,23; figures 6-9; example 5 *<br>--- | 1,2,<br>5-14,16,<br>21-24,<br>26,27 | C12N15/00<br>A61K48/00<br>C12N9/12<br>C12N15/38<br>A01K67/00 |
| X | WO-A-9 007 936 (CHIRON CORPORATION)<br><br>* the whole document *<br>--- | 1,2,<br>5-14,16,<br>21-24,<br>26,27 | |
| X | EP-A-0 370 813 (TRANSGENIC SCIENCES INC.)<br><br>* the whole document *<br>--- | 1-3,6-9,<br>11,12,<br>25-27 | |
| P,X | EP-A-0 415 731 (THE WELLCOME FOUNDATION)<br><br>* the whole document *<br>----- | 1,2,<br>5-12,15,<br>21-23,<br>26,27 | TECHNICAL FIELDS SEARCHED (Int. Cl.5 )<br><br>C12N<br>A61K |

The present search report has been drawn up for all claims

| Place of search | Date of completion of the search | Examiner |
|---|---|---|
| THE HAGUE | 08 APRIL 1992 | CHAMBONNET F.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................................
& : member of the same patent family, corresponding
    document

EPO FORM 1503 03.82 (P0401)